# EUROPÄISCHE PATENTSCHRIFT

(11) **EP 0 545 350 B1**
(45) Veröffentlichungstag und Bekanntmachung des Hinweises auf die Patenterteilung: **03.07.1996**
(21) Anmeldenummer: 92120471.5
(22) Anmeldetag: 01.12.1992
(51) Int. Cl.: G01N 33/531, G01N 33/548

(54) **Multivalentes Dextranreagenz zum Einsatz in Präzipitationstests**
Multivalent dextran-reagent used in precipitation tests
Réactif multivalent à base de dextrane utilisé dans les essais de précipitation

(30) Priorität: 05.12.1991 DE 4140142
(43) Veröffentlichungstag der Anmeldung: 09.06.1993
(73) Patentinhaber: BOEHRINGER MANNHEIM GMBH, D-68298 Mannheim (DE)
(72) Erfinder: Karl, Johann, Dr., W-8123 Peissenberg (DE); Maier, Josef, W-8120 Weilheim (DE)

(56) Entgegenhaltungen:
- EP-A- 0 405 578
- EP-A- 0 483 512
- WO-A-82/04323
- FR-A- 2 601 455
- US-A- 4 530 900

## Beschreibung

Die Erfindung betrifft ein multivalentes Dextranreagenz zum Einsatz in einem Präzipitationstest zur Bestimmung einer spezifisch bindefähigen Substanz, bestehend aus Dextran, an das mehrere Moleküle eines Rezeptors R₁, der mit der zu bestimmenden Substanz spezifisch bindefähig ist, oder der spezifisch bindefähige Substanz bzw. eines Analogons dieser Substanz gebunden sind.

Weiterer Gegenstand ist ein Präzipitationstest zur Bestimmung einer spezifisch bindefähigen Substanz, bei dem neben den üblicherweise für eine Präzipitationsreaktion notwendigen Reagenzien, wie Puffer, Entstörsubstanz, Reaktionsbeschleuniger oder Detergenz das multivalente Dextranreagenz eingesetzt wird.

In Körperflüssigkeiten und Geweben kommen sehr viele Substanzen vor, die mit einem spezifischen Bindungspartner bindefähig sind und als Parameter für bestimmte Erkrankungen oder den Gesundheitszustand des Körpers dienen. Hierunter zählen u. a. Haptene, wie z. B. Hormone, Proteine, wie z. B. C-reaktives Protein (CRP), glykierte Proteine und virale Proteine sowie Antikörper. Bei der Überwachung einer medikamentösen Behandlung ist oft die Bestimmung von Arzneistoffen im Blut erforderlich. Die bindefähigen Substanzen kommen in sehr unterschiedlichen Konzentrationen in den Körperflüssigkeiten oder Geweben vor. Verschiedene Proteine, wie z. B. IgG, IgA oder Apolipoproteine kommen in hohen Konzentrationen vor, während z. B. Hormone oder Arzneistoffe aber auch andere Proteine in sehr niedrigen Konzentrationen vorliegen können. Der Nachweis dieser Substanzen kann durch einen Präzipitationstest erfolgen. Spezifisch bindefähige Substanzen, die mindestens bivalent sind, d. h. mindestens zwei Epitope besitzen, die mit dem Rezeptor im Präzipitationstest binden, können direkt durch die Zugabe eines spezifischen bindefähigen Rezeptors präzipitiert werden. Zur Erhöhung der Empfindlichkeit der nephelometrischen oder turbidimetrischen Messung ist es oft erforderlich, die spezifisch bindefähigen Rezeptoren an hochpolymere Teilchen, z. B. Latex oder rote Blutkörperchen, zu binden. Solche als Agglutinationstests bezeichnete Nachweismethoden mit turbidimetrischer Auswertung wurden in Eur. J. Biochem. 20 (1971), 553-560 beschrieben.

Niedrigmolekulare Substanzen, die nur ein Epitop besitzen, an das der spezifische Rezeptor im Präzipitations- oder Agglutinationstest bindet, wie z. B. Haptene, lassen sich nicht mit der direkten Testführung des Präzipitations- oder Agglutinationstests nachweisen, da sich keine Quervernetzung ausbilden kann, die zur Präzipitation führt. Aus der EP-A-0 079 962 ist ein Immuno-Präzipitationstest, der eine kompetitive Testführung nutzt, bekannt, bei dem die die zu bestimmende Haptene enthaltende Lösung mit einem haptenbeschichteten Albumin in Kontakt gebracht wird. Durch Zugabe von mit dem Hapten bindefähigen Antikörpern erfolgt eine Präzipitationsreaktion. Da das an das Albumin gebundene Hapten mit dem in der Probe enthaltenen Hapten konkurriert, ist die Präzipitationsreaktion umso geringer, je mehr Hapten in der Probe enthalten ist. Als Haptenträger sind im Stand der Technik noch andere Proteine, z. B. IgG, Latexteilchen oder synthetische Polymere, wie z. B. Polyaspartat (EP-A-0 317 796) beschrieben.

Alle diese Agglutinations- oder Präzipitationstests besitzen teilweise noch erhebliche Nachteile. Reine Präzipitationsteste besitzen für einige Parameter eine zu geringe Sensitivität. Deshalb werden spezifische Rezeptoren zur Erhöhung der Empfindlichkeit in Agglutinationstests an Latexteilchen gebunden. Durch die Kopplung kann einerseits die Reaktivität des Rezeptors selbst beeinträchtigt werden, andererseits kann die Zugabe von Reaktionsbeschleunigern, wie z. B. Polyethylenglykol, die zur Vermeidung einer langen Inkubationsperiode zugesetzt werden müssen, zu spontanen Agglutinationsreaktionen der Latexteilchen führen. Auch ist es schwierig, die Anzahl der an die einzelnen Latexpartikel gekoppelten Rezeptoren exakt zu steuern. Zwischen den einzelnen Chargen können deshalb erhebliche Schwankungen auftreten. Die Bindung von unspezifischen Faktoren aus der Probe an die Oberfläche der Latexpartikel kann zu Meßungenauigkeiten führen.

Bei dem Einsatz von trägergebundenen Haptenen, wie Hapten-Albumin oder Hapten-IgG, können ebenfalls Schwierigkeiten auftreten. Mögliche Störfaktoren sind anti-IgG und anti-Albumin Antikörper im Serum. Auch hier ist es schwierig, die Anzahl der Haptene an den einzelnen Molekülen, vor allem bei der Verwendung von natürlichen Molekülen, wie Proteine, exakt zu kontrollieren. Darüber hinaus sind diese Materialien nicht in einem Maße lagerstabil, der ihren unbegrenzten Einsatz erlaubt. Diese Materialien sind bezüglich ihres Lösungsverhaltens und ihrer Denaturierungstendenz nicht optimal und daher nicht universell einsetzbar.

Bisher war es erforderlich, je nach Testführung, wobei zwischen direkter oder kompetitiver Testführung unterschieden wird, nachzuweisender Substanz, wie Haptene, Antigene oder Antikörper, und Konzentration der nachzuweisenden Substanz das optimale Trägermaterial zur Bindung des spezifischen Rezeptors oder der spezifisch bindefähigen Substanz für einen Agglutinationstest oder einen Präzipitationstest mit trägergebundenen Haptenen aus den möglichen zur Verfügung stehenden Materialien jeweils auszutesten. Ein Trägermaterial, das universell bei allen Testvarianten, nachzuweisenden Substanzen und Konzentrationen eingesetzt werden kann und außerdem eine hohe Lagerstabilität, ein gutes Lösungsverhalten und eine geringe Denaturierungstendenz aufweist sowie den Einsatz verschiedener Molekülgrößen und unterschiedlichster Einbauraten ermöglicht, ist bisher nicht bekannt.

Aufgabe der vorliegenden Erfindung war es daher, ein Trägermaterial zum universellen Einsatz bei direkten oder kompetitiven Präzipitationstests zur Verfügung zu stellen, das den Nachweis von Substanzen, die eine oder mehrere Bindungsstellen aufweisen, mit hoher Empfindlichkeit, Genauigkeit und Stabilität ermöglicht und die oben beschriebenen Nachteile nicht hat.

Die Aufgabe wird durch die in den Ansprüchen näher charakterisierte Erfindung gelöst. Im wesentlichen wird diese Aufgabe gelöst durch ein multivalentes Dextranreagenz zum Einsatz in einem Präzipitationstest zur Bestimmung einer spezifisch bindefähigen Substanz, bestehend aus Dextran, an das mehrere Moleküle eines Rezeptors R₁, der mit der zu bestimmenden Substanz spezifisch bindefähig ist, oder der spezifisch bindefähige Substanz bzw. eines Analogons dieser Substanz gebunden sind.

Das multivalente Dextranreagenz findet Verwendung in einem Präzipitationstest zur Bestimmung einer spezifisch bindefähigen Substanz.

Ein weiterer Gegenstand der Erfindung ist ein Präzipitationstest sowie ein Testkit zur Bestimmung einer spezifisch bindefähigen Substanz, in dem das multivalente Dextranreagenz neben weiteren für das Verfahren notwendigen Hilfs- und Zusatzstoffen eingesetzt wird bzw. enthalten ist.

Der das multivalente Dextranreagenz enthaltende Präzipitationstest ist zur Bestimmung vieler in Körperflüssigkeiten oder Gewebeextrakten nachzuweisenden zu einer spezifischen Bindung befähigten Substanzen geeignet, wobei niedrig konzentrierte Substanzen ebenso nachweisbar sind, wie hoch konzentrierte. Sowohl mono- als auch polyvalente Substanzen können bestimmt werden. Bevorzugt wird das multivalente Dextranreagenz zur Bestimmung von monovalenten Substanzen eingesetzt. Da im Serum keine gegen das multivalente Dextranreagenz gerichteten Antikörper vorkommen sollten, treten keine hierauf zurückzuführenden Antikörperstörungen bei Verwendung des Dextranreagenzes im Test auf.

Als monovalent wird dabei eine Substanz bezeichnet, die für einen spezifischen Partner nur eine Bindungsstelle besitzt. Als Beispiele sind hier Haptene, z. B. Hormone, Peptide, wie glykiertes Hämoglobin (HbA₁), und Arzneimittel zu nennen. Als polyvalent wird die Substanz bezeichnet, wenn sie mindestens zwei Bindungsstellen für den spezifisch bindefähigen Bindungspartner besitzt, wie z. B. Proteine, wie CRP und α-1 Mikroglobulin, glykierte Proteine, wie glykiertes Albumin und glykiertes IgG, Antigene und Antikörper wie IgG, Rheumafaktor (RF) oder Antistreptolysin O (ASLO).

Die Probelösung wird dazu mit einem multivalenten Dextranreagenz versetzt, an das entweder ein Rezeptor R₁, der mit der nachzuweisenden Substanz spezifisch bindefähig ist, oder die spezifisch bindefähige Substanz bzw. ein Analogon dieser Substanz gebunden ist. Unter multivalentem Dextranreagenz werden Dextranmoleküle verstanden, an die mehrere Moleküle des Rezeptors R₁ oder der spezifisch bindefähigen Substanz bzw. dem Analogon dieser Substanz gebunden sind. Zusätzlich werden zu dem Testansatz weitere für einen Präzipitationstest notwendige Reagenzien, wie Puffer, Reaktionsbeschleuniger, Entstörsubstanz, Detergenz und andere zugesetzt, wobei die Zugabe dieser Reagenzien zur Probe nicht unbedingt gleichzeitig mit dem Dextranreagenz erfolgen muß.

Es sind verschiedene Reaktionsprinzipien möglich, die mit dem Verfahren, das das erfindungsgemäße multivalente Dextranreagenz verwendet, durchgeführt werden können.

Die Variante 1 dient zum Nachweis bi- oder polyvalenter Substanzen, die mindestens zwei Bindungsstellen für den mit dieser Substanz spezifisch bindefähigen Rezeptor R₁ besitzen. Es wird eine direkte Testführung benutzt, wobei ein multivalentes Dextranreagenz eingesetzt wird, bestehend aus Dextran, an das mehrere Moleküle eines Rezeptors R₁, der mit der zu bestimmenden Substanz spezifisch bindefähig ist, gebunden sind. Das Ausmaß der Präzipitationsreaktion ist direkt proportional der in der Probe zu bestimmenden Substanz.

Die Variante 2 dient ebenso zum Nachweis polyvalenter Substanzen, wobei auch die direkte Testführung benutzt wird. In diesem Fall ist der Rezeptor R₁, der mit der zu bestimmenden Substanz spezifisch bindefähig ist, nicht direkt an Dextran gebunden, sondern wird über zwei miteinander bindefähige Rezeptoren R₃ und R₄ gebunden. R₃ ist dabei an das Dextranmolekül und R₄ an R₁ gebunden.

Mit der dritten Variante werden bevorzugt monovalente Substanzen, wie Haptene, kurze Peptide oder Arzneimittel, nachgewiesen. Das Prinzip entspricht dem eines kompetitiven Präzipitationstests. Das multivalente Dextranreagenz besteht in diesem Falle aus Dextran, an das die spezifisch bindefähige Substanz bzw. ein Analogon dieser Substanz gebunden ist. Zu dem Testansatz wird ein mindestens bivalenter Rezeptor R₂, der mit der zu bestimmenden Substanz spezifisch bindefähig ist, zugesetzt. Bei Inkubation der Probelösung mit dem multivalenten Dextranreagenz und R₂ konkurriert die zu bestimmende Substanz aus der Probe und das multivalente Dextranreagenz um die Bindung an R₂. Das Ausmaß der Präzipitation ist umgekehrt proportional zu der in der Probe nachzuweisenden Substanz.

Die Variante 4 entspricht im Prinzip der Variante 3, mit dem Unterschied, daß die spezifisch bindende Substanz bzw. ein Analogon dieser Substanz nicht direkt an Dextran gebunden ist, sondern über die Rezeptoren R₃ und R₄, gebunden werden kann, wobei R₃ an das Dextranmolekül und R₄ an die spezifisch bindefähige Substanz bzw. einem Analogon dieser Substanz gebunden ist.

Die Verwendung der Rezeptoren R₃ und R₄ bei den Testvarianten 2 und 4 hat den Vorteil, daß das Dextranmolekül, an das der Rezeptor R₃ gekoppelt ist, universell bei mehreren nachzuweisenden Parametern eingesetzt werden kann.

Durch den Einsatz des erfindungsgemäßen multivalenten Dextranreagenzes in direkten oder kompetitiven Präzipitationstests wird die Empfindlichkeit des Nachweises der spezifisch bindefähigen Substanz im Vergleich zu herkömmlichen Präzipitationstests erhöht. Eine Steigerung der Empfindlichkeit durch den Einsatz von Partikeln, wie z. B. Latexpartikeln, erscheint meist nicht mehr erforderlich.

Bestimmte niedrig konzentrierte Parameter erfordern eine sehr niedrige Nachweisgrenze, z. 3. niedrig dosierte Arzneimittel. Die Empfindlichkeit des erfindungsgemaßen kompetitiven Präzipitationstests - die oben beschriebenen Varianten 3 und 4 - kann noch weiter gesteigert werden, indem in der als Testvariante 5 bezeichneten Ausführung zusätzlich zu dem multivalenten Dextranreagenz, bestehend aus Dextran, an das mehrere Moleküle der spezifisch bindefähigen Substanz bzw. eines Analogons dieser Substanz gebunden sind ein Rezeptor R₂ eingesetzt wird, von dem ebenfalls mehrere Moleküle an Dextran gebunden sind. Hierbei ist es erforderlich, die Beladung der Dextranreagenzien mit der spezifisch bindefähigen Substanz und den Rezeptor R₂ genau zu steuern und aufeinander abzustimmen. Das erfindungsgemäße multivalente Dextranreagenz bietet hierfür vorzügliche Voraussetzungen, da die Beladung sehr genau gesteuert werden kann. Es werden also in der Testvariante 5 zwei multivalente Dextranreagenzien, die miteinander zu einer Präzipitationsreaktion befähigt sind, eingesetzt. Durch Zugabe der zu bestimmenden Substanz wird diese Präzipitationsreaktion vermindert.

Für den erfindungsgemäß definierten Präzipitationstest gibt es also mehrere Durchführungsvarianten. Die zu bestimmende Substanz kann jede zu einer spezifischen Bindung fähige Substanz sein und insbesondere - wie oben definiert - ein Hapten, ein monovalentes, bivalentes oder polyvalentes Antigen oder ein Antikörper sein.

Das Grundgerust des Dextranreagenzes bildet das Polysaccharid Dextran. Dieses ist in verschieden hohen Polymerisationsgraden verwendbar. Als besonders geeignet erwiesen hat sich Dextran mit einem Molekulargewicht von 10000 bis zur Löslichkeitsgrenze, die bei etwa 2 Millionen liegt. Vorzugsweise wird Dextran mit einem Molekulargewicht von 20000 bis 500000 eingesetzt. Innerhalb dieser bevorzugten Grenzen besitzt der erfindungsgemäße Präzipitationstest die höchste Sensivität.

Die Bezeichnung "multivalentes Dextranreagenz" bedeutet, daß an einem Dextranmolekül mehrere Moleküle des Rezeptors R₁ oder der spezifisch bindefähigen Substanz bzw. deren Analogon gebunden sind. Das Verhältnis Dextran : gebundene Moleküle wird als Beladung bezeichnet. Als geeignet hat sich eine Beladung von 1 : 2 bis 1 : 50 erwiesen. Besonders geeignet sind Beladungen von 1 : 5 bis 1 : 40. Bei noch dichterer Beladung der Dextranmoleküle können Störungen durch sterische Effekte auftreten. Die Einbaurate ist innerhalb der genannten Grenzen für jede bestimmte bindefähige Substanz zu optimieren. Je nach der Konzentration der nachzuweisenden bindefähigen Substanz in der Probe sind unterschiedliche Einbauraten innerhalb der genannten Grenzen vorteilhaft. Im Vergleich zu den bisher im Stande der Technik meist verwendeten Trägermolekülen, wie RSA und IgG zeigte sich, daß die Beladung bei dem Dextranreagenz höher liegen kann. Hierdurch wird im Test eine Steigerung der Sensitivität bewirkt.

Als Rezeptor R₁ wird ein Molekül gewählt, das mit der zu bestimmenden Substanz spezifisch bindefähig ist. Der Rezeptor R₁ und die zu bestimmende Substanz stellen also ein Bindungspaar dar. R₁ muß mindestens eine Bindungsstelle aufweisen, kann aber auch zwei oder mehrere Bindungsstellen für die zu bestimmende Substanz besitzen. Da mehrere Rezeptoren an dem Dextranreagenz gebunden sind , führt ein Rezeptor R₁ mit nur einer Bindungsstelle zu einer Vernetzung und damit zu einer Präzipitation. Der Rezeptor R₁ wird nach der jeweils zu bestimmenden Substanz ausgewählt. Hier sind eine Vielzahl von Rezeptoren geeignet. Zur Bestimmung von Antigenen, Proteinen, DNA oder Zucker ist es besonders vorteilhaft, Antikörper oder Antikörperfragmente, wie F(ab)₂, Fab oder Fab'-Fragmente zu verwenden. Zur Bestimmung von DNA ist es ebenfalls vorteilhaft, eine komplementäre DNA als Rezeptor R₁ zu verwenden. Soll in der Probe selbst ein Antikörper oder ein anderer Probenrezeptor, der mindestens zwei Bindungsstellen aufweist, bestimmt werden, so kann als Rezeptor R₁ der komplementäre Bindungspartner, wie z. B. das Hapten, Antigen, Protein, Zucker oder auch Fragmente davon, wie z. B. einzelne Epitope eines Proteins, verwendet werden.

Bei der kompetitiven Testvariante wird die spezifisch bindefähige Substanz bzw. ein Analogon dieser Substanz an Dextran direkt oder über die Rezeptoren R₃ und R₄ gebunden. Die spezifisch bindefähige Substanz kann bevorzugt der unveränderten zu bestimmenden Substanz entsprechen. Es kann auch ein Derivat der zu bestimmenden Substanz bzw. ein Teil der zu bestimmenden Substanz, wie beispielsweise ein Proteinepitop, verwendet werden. Wesentlich ist nur, daß die Substanz bzw. das Derivat oder Teil mit dem Rezeptor R₂ bindefähig ist, wobei es nicht unbedingt erforderlich ist, daß der Rezeptor R₂ die gleichen Bindungsstärken zu diesen gebundenen Substanzen und zu der in der Probe vorliegenden zu bestimmenden Substanz aufweist.

Als Rezeptor R₂, der bei der kompetitiven Testführung der Testvarianten 3 und 4 zusätzlich zum multivalenten Dextranreagenz benötigt wird, können alle Moleküle eingesetzt werden, die mit der zu bestimmenden Substanz spezifisch bindefähig sind. Sie entsprechen dem Rezeptor R₁ mit der Ausnahme, daß der Rezeptor R₂ mindestens zwei Bindungsstellen aufweisen muß. Rezeptoren mit nur einer Bindungsstelle für die spezifisch bindefähige Substanz, wie z. B. Fab oder Fab'-Fragmente, können nicht verwendet werden.

Bei der Testvariante 5, bei der der Rezeptor R₂ an Dextran gebunden eingesetzt wird, können dagegen neben bi- und polyvalenten Rezeptoren auch monovalente Rezeptoren eingesetzt werden. Es können damit alle Rezeptoren verwendet werden, die als Rezeptor R₁, wie oben dargestellt, verwendet werden können. Da mit der kompetitiven Testführung bevorzugt Haptene nachgewiesen werden, werden als Rezeptoren bevorzugt die spezifischen Antikörper oder Antikörperfragmente verwendet.

Zur Bindung des Rezeptors R1 oder der spezifisch bindefähigen Substanz bzw. dem Analogon dieser Substanz werden bei den Testvarianten 2 und 4 die miteinander bindefähigen Rezeptoren R₃ und R₄ genutzt, wobei R₃ an Dextran und R₄ an R₁ oder die spezifisch bindefähige Substanz bzw. das Analogon dieser Substanz gebunden ist. Geeignete Bindungspaare R₃-R₄ sind insbesondere Biotin-Streptavidin bzw. Avidin, Hapten-Antikörper, Antigen-Antikörper, Concanavalin-Antikörper, Zucker-Lectin, Hapten-Bindeprotein, z. B. Thyroxin und Thyroxinbindendes Globulin, oder Oligopeptid-Antikörper.

Bevorzugt wird als bindendes Paar Streptavidin bzw. Avidin-Biotin eingesetzt, wobei besonders bevorzugt an Dextran Streptavidin bzw. Avidin gebunden wird. Biotin wird bevorzugt an den Rezeptor R₁ oder die spezifisch bindefähige Substanz bzw. an das Analogon dieser Substanz gebunden. Die Herstellung dieses Biotin-Konjugats erfolgt nach an sich bekannten Methoden (z. B. analog European Journal of Biochemistry 131 (1980) 333-338).

Die Bindung des Rezeptors R₁, der spezifisch bindefähigen Substanz bzw. des Analogons dieser Substanz oder des Rezeptors R₃ an Dextran erfolgt nach bekannten Methoden des Standes der Technik. Die Kopplung kann direkt an den im Dextran vorhandenen Hydroxylgruppen erfolgen. Bevorzugt -werden zur Kopplung in das Dextranmolekül weitere funktionelle Gruppen, wie Amino-, Carboxyl-, Sulfhydryl-, Chloromethyl-, Hydrazido- oder Diazoniumreste eingeführt.

Besonders bevorzugt werden als funktionelle Gruppen Aminoreste eingefügt. An Dextran bzw. funktionalisiertes Dextran wird der Rezeptor R₁, die spezifisch bindefähige Substanz bzw. das Analogon dieser Substanz oder der Rezeptor R₃ mit Hilfe von Kopplungsreagenzien, wie beispielsweise in der EP-A-0 317 796 beschrieben, gekoppelt. Die Beladung wird über das Stöchiometrieangebot und die Reaktionsbedingungen, wie pH, Temperatur und Reaktionszeit gesteuert.

In dem erfindungsgemäßen Präzipitationstest können noch weitere Hilfs- oder Zusatzstoffe, z. B. als Reaktionsbeschleuniger enthalten sein. Als Reaktionsbeschleuniger wird üblicherweise PEG 6000 in Konzentrationen von 1 - 5 Gew.% zugesetzt. Weiterhin können Detergenzien in Konzentrationen zwischen 0,01 - 4 Gew.% enthalten sein.

Das Verfahren kann in einer oder mehreren Stufen durchgeführt werden. Die Auswertung erfolgt durch Messung des Ausmaßes der Präzipitation. Verfahren hierzu sind bekannt. Geeignet ist beispielsweise die photometrische Trübungsmessung oder die Streulichtmessung durch Nephelometrie.

Ein weiterer Gegenstand der vorliegenden Erfindung ist ein Testkit zur Bestimmung von spezifisch bindefähigen Substanzen, der neben weiteren für den Präzipitationstest notwendigen Hilfs- oder Zusatzstoffen ein multivalentes Dextranreagenz, bestehend aus Dextran, an das mehrere Moleküle des Rezeptors R₁, der mit der zu bestimmenden Substanz spezifisch bindefähig ist, oder der spezifisch bindefähigen Substanz bzw. eines Analogons dieser Substanz gebunden sind , enthält.

Als Hilfs- oder Zusatzstoffe können im Testkit neben den üblicherweise eingesetzten Puffersubstanzen, Reaktionsbeschleuniger in Konzentrationen von 1 - 5 %, Detergenzien in Konzentrationen von 0,01 - 4 % oder Entstörsubstanzen enthalten sein. Bei den Testkits, die die Reagenzien für die Verfahrensvarianten 3 und 4 enthalten, sind zusätzlich die zur Präzipitationsreaktion benötigten Rezeptoren R₂ enthalten.

Die Erfindung wird durch die folgenden Beispiele erläutert.

### Beispiel 1: Nachweis von Phenobarbital (PHEBA)

### 1.1 Herstellung von Aminodextran

11,6 g Chloressigsäure, Natriumsalz werden in 100 ml H₂O gelöst, zu einer Lösung aus 11,2 g Dextran mit einem Molekulargewicht von 40000 in 36 ml 1 M NaOH gegeben und 20 Stunden bei 40 °C gerührt. Danach wird mit 1 M HCl ein pH-Wert von 4 eingestellt und am Rotationsverdampfer auf ein Volumen von 50 ml konzentriert. Das gebildete Carboxymethyl-Dextran wird in 50 ml 2 M Ethylendiamin x 2 HCl, pH 5, gelöst und portionsweise innerhalb 60 Minuten mit 3 g N-Ethyl-N'(3-dimethylamino-propyl)carbodiimid x HCl versetzt. Bei einem konstanten pH-Wert von 4,7, wobei NaOH bzw. HCl mittels eines Titrators zugegeben wird, wird bei Raumtemperatur drei Stunden weitergerührt. Das Produkt wird zweimal gegen entsalztes H₂O dialysiert und anschließend lyophilisiert.

### 1.2 Herstellung von Phenobarbital (PHEBA)-Dextran

An Aminodextran mit einem Molekulargewicht von 40000 wurde das Hapten Phenobarbital gekoppelt. Als Linker wurde 1-Carboxypropyl-ortho-Succinimid (1-cp-osu) benutzt. Nach Herstellung von PHEBA-1-cp-osu wurde dieses an die Aminogruppen des Aminodextrans durch Umaminidierung nach Anderson, G.W. et al., J. Amer. Chem. Soc. 86 (1964), 1839 gekoppelt. Um eine Beladung von 1 : 10 (Dextran : PHEBA) zu erhalten, wurden 400 mg Aminodextran in 40 ml 50 mM KHPO₄-Puffer, pH 8,5 mit 100 mM NaCl gelöst und 41,5 mg PHEBA-1-cp-osu in 4,15 ml Dioxan tropfenweise zugefügt. Nach zwei Stunden Reaktion bei Raumtemperatur wurde mit 2 ml 0,1 M Lysinlösung pH 8,5 abgestoppt. Das PHEBA(-1-cp-osu)-Dextran wurde 3mal gegen das 2000fache Volumen dialysiert, um Verunreinigungen zu entfernen.

### 1.3 Präzipitationstest zum Nachweis von Phenobarbital

Folgende Lösungen wurden verwendet:

### Lösung 1: (Reaktionspuffer mit Dextranreagenz)

200 µg/ml PHEBA(-i-cp-osu)-Dextran
mit einer Beladung von 1 : 10
100 mM KPO₄, pH 7,4
4 % Polyethylenglykol (PEG) 40000
0,1 % Rinderserumalbumin
1 % Tween-20®
0,1 % NaN₃

### Lösung 2: (Antiserum)

Polyklonales Schaf-Antikörper-Rohserum gegen PHEBA wurde mit 100 mM KPO₄, pH 7,4, 1 : 10 verdünnt. Die Einsatzmenge pro Test betrug 5 µl Rohserum. Dies entspricht etwa 100 µg polyklonale Antiköper gegen PHEBA.

### Lösung 3: (Probe)

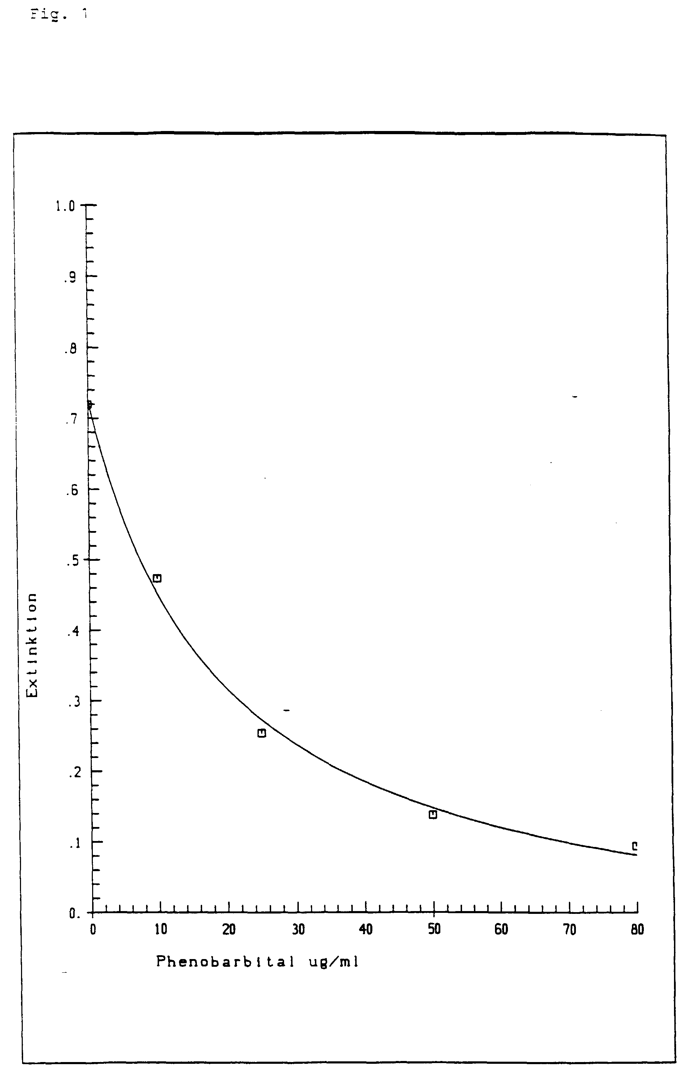

Als Standard wurde Humanserum, das mit 0-80 µg PHEBA/ml angereichert wurde, eingesetzt. Die Messung wurde an einem Hitachi 704 der Firma Boehringer Mannheim GmbH (Mannheim, Deutschland) bei einer Temperatur von 30° C, einer Meßwellenlänge von 340 nm und einer Korrekturwellenlänge von 700 nm durchgeführt. 10 µl Lösung 3 (Probe) wurden mit 350 µl Lösung 1 vermischt und 5 Minuten inkubiert. Danach erfolgte die Messung der Extinktion El. 50 µl Lösung 2 wurden zupipettiert und der Testansatz weitere 5 Minuten inkubiert. Danach erfolgte die zweite Extinktionsmessung E2. Zur Auswertung der Ergebnisse wurde die Extinktionsdifferenz Δ E = E2-E1 gegen die PHEBA-Konzentration graphisch aufgetragen. Die Ergebnisse des kompetitiven Nachweises von PHEBA sind in Fig. 1 graphisch dargestellt.

### Beispiel 2

### Bestimmung von glykosyliertem Hämoglobin (HbA_{1c})

Zur Herstellung des multivalenten Dextranreagenzes mit gekoppeltem HbA_{1c} wurde von Aminodextran (MG 40000) ausgegangen, das entsprechend der Angaben unter 1.1 hergestellt wurde. Die Kopplung des glykosylierten Hämoglobins bzw. eines HbA_{1c}-Analogons erfolgte über dessen Sulfhydrylgruppe an Maleimido-funktionalisiertes Aminodextran nach Kitagawa et al., J. Biochem. 79 (1976), 233.

Als HbA_{1c}-Analogon wurde das fructosylierte N-terminale Tetrapeptid der β-Kette des Hämoglobins verwendete Das Dextran-Peptid-Konjugat (Fruc-1-4(Cys, MHS)-Dextran) wurde mit Beladungsdichten von 1 : 14 und 1 : 28 (Dextran : Peptid) hergestellt. Als Vergleich zur Methode des Standes der Technik wurde analog ein Peptid-RinderserumalbuminKonjugat (Fruc-1-4(Cys, MHS)-RSA) mit einer Beladungsdichte von 1 : 18 hergestellt.

Um eine Beladungsdichte von 1:14 bei Dextran zu erreichen, wurden 50 mg Aminodextran in 5 ml 50 mM KPO₄-Puffer, pH 6,8 gelöst und bei Raumtemperatur unter Rühren mit einer Lösung aus 10 mg Maleimidohexanoyl-N-hydroxy-succinimidester (MHS) in 0,5 ml Dioxan tropfenweise versetzt. Nach zwei Stunden Reaktionszeit wird das Reaktionsprodukt gegen 5 Liter 20 mM KPO₄-Puffer, pH 7,0 dialysiert. Unter diesen Bedingungen wird reproduzierbar eine Beladung von 1:14 erreicht.

50 mg aktiviertes Aminodextran in 2,5 ml KPO₄-Puffer, pH 6,8 mit einer Lösung aus 137 mg HbA_{1c}-Cys-Peptid in 1 ml 100 mM KPO₄-Puffer, pH 6,8 bei Raumtemperatur versetzt. Nach zwei Stunden Reaktionszeit wird der Ansatz über ACA 202 chromatographisch abgetrennt.

Um eine Beladung von 1:28 zu erreichen, wurden 400 mg Aminodextran in 20 ml 50 mM KHPO₄-Puffer, pH 6,8 gelöst und entsprechend den obigen Angaben mit 140 mg MHS in 12,3 ml Dioxan versetzt und zur Reaktion gebracht.

480 mg aktiviertes Aminodextran wurden in 20 ml 100 mM KHPO₄-Puffer, pH 6,8 mit einer Lösung aus 274 mg HbA_{1c}-Cys-Peptid in 4,22 ml KHPO₄-Puffer zwei Stunden bei Raumtemperatur inkubiert und das Reaktionsprodukt chromatographisch über ACA abgetrennt.

Entsprechend diesen Angaben wurde Rinderserumalbumin mit MHS aktiviert und daran das HbA_{1c}-Cystein-Peptid gekoppelt.

Der Präzipitationstest wurde entsprechend der Angabe unter 1.3 an einem Hitachi 704 der Firma Boehringer Mannheim GmbH durchgeführt.

Folgende Lösungen wurden eingesetzt:

### Lösung 1: (Antiserum)

20 mM MES, pH 6,0
150 mM NaCl
0,5 % Detergenz
3 % PEG 6000
6,0 mg/ml PAK 〈HbA₁〉-S-IgG (DE) bzw.
5,0 mg/ml PAK 〈HbA_{1c}〉-S-IgG (DE)

### Lösung 2: (Dextranreagenz)

20 mM MES, pH 6,0
150 mM NaCl
0,5 % Detergenz
6,0 % PEG 6000
25 µg/ml Fruc-1-4(Cys, MHS)-Dextran 1 : 14
bzw. 20 µg/ml Fruc-1-4(Cys, MHS)-Dextran 1 : 28
bzw. 25 µg/ml Fruc-1-4(Cys, MHS)-RSA 1 : 18

### Lösung 3:

Zur Eichkurvenerstellung diente hämolysiertes und denaturiertes EDTA-Blut mit bekanntem HbA_{1c}-Gehalt, das mit Hämolysereagenz entsprechend verdünnt wurde. Zur Hämolyse und Denaturierung können bekannte Verfahren nach dem Stand der Technik verwendet werden.

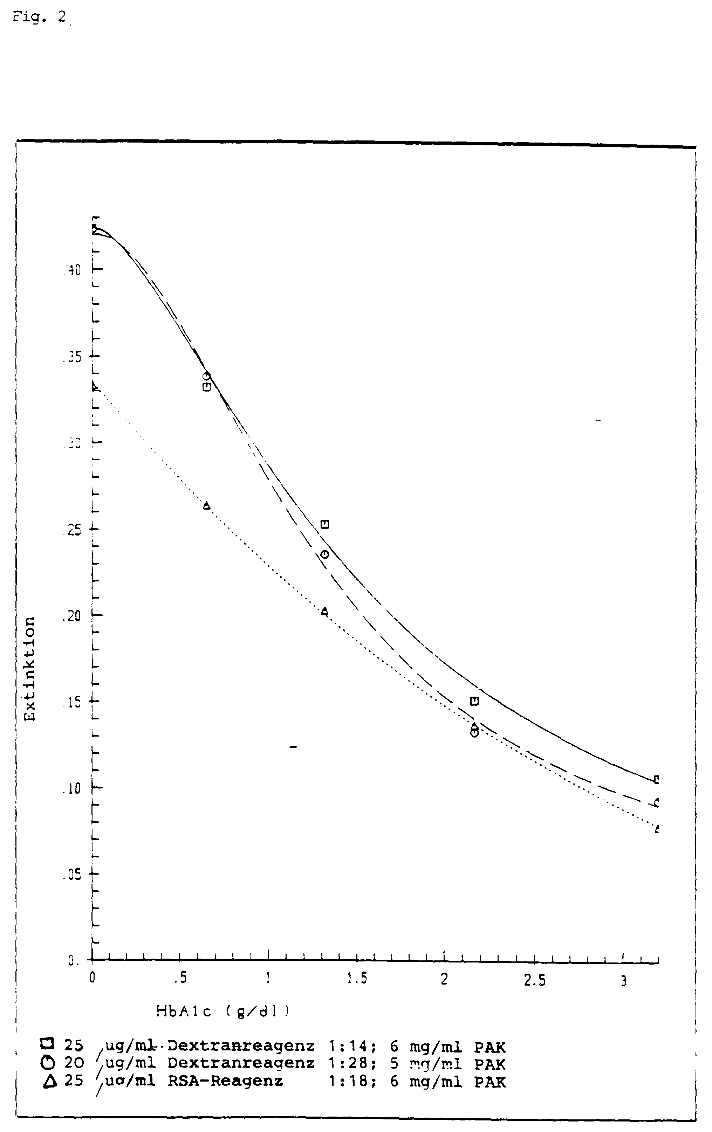

Bei 37° C wurden 250 µl Lösung 1 und 6 µl Lösung 3 5 Minuten inkubiert. Danach wurde die Extinktion E1 bei 340 nm (Korrekturwellenlänge 700 nm) gemessen. Nach der Zugabe von 50 µl Lösung 2 und weiteren 5 Minuten Inkubation wurde die Extinktion E2 bestimmt. Zur Auswertung wurde die Extinktionsdifferenz Δ E = E2-E1 gegen die HbA_{1c}-Konzentration graphisch dargestellt (Fig. 2). Mit dem erfindungsgemäßen Dextranreagenz wird unabhängig von der Beladungsdichte eine höhere Empfindlichkeit wie mit einem RSA-Reagenz mit einer Beladungsdichte von 1 : 18 erzielt, wobei ein höherer Einbau bei RSA nicht mehr möglich ist. Bei Verwendung des Dextranreagenzes mit der Beladungsdichte von 1 : 28 führt bereits der Einsatz von weniger PAK und Dextranreagenz zur gleichen Eichkurve wie das niedrig beladene Dextranreagenz. Der Einsatz höherer Konzentrationen (c_{PAK} = 6 mg/ml; C_{Dextranreagenz} = 25 µg/ml) mit dem Dextranreagenz 1:28 führt zu weiterer Steigerung der Empfindlichkeit des kompetitiven Immunoassays.

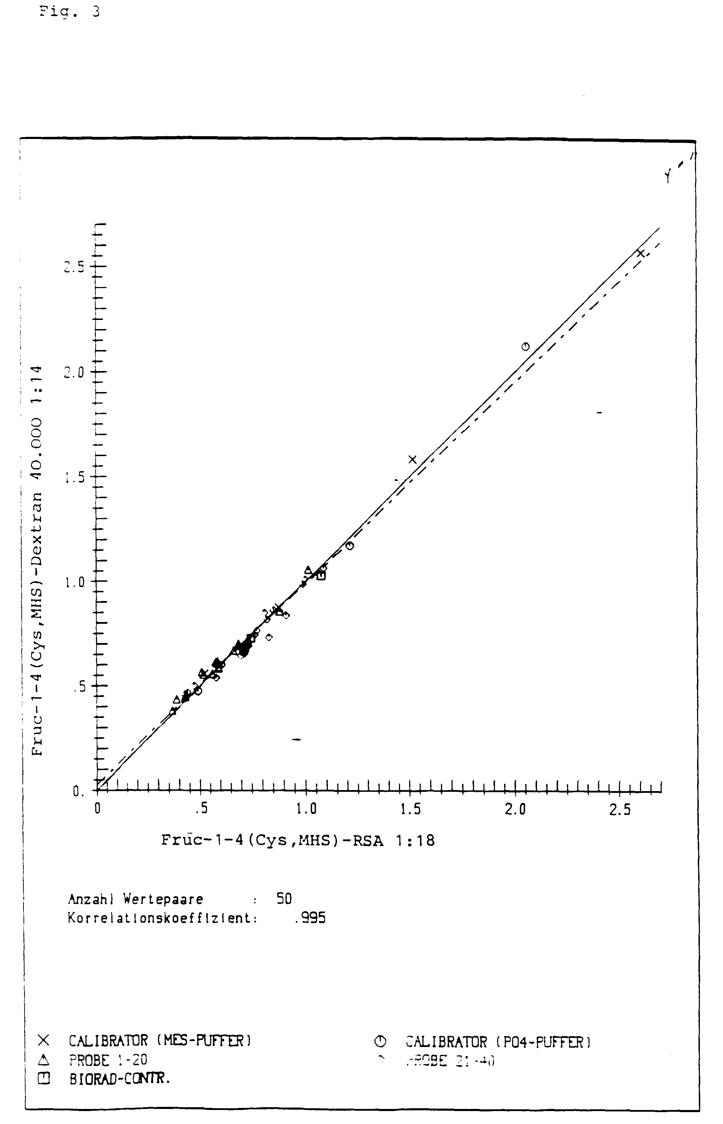

Bei dem Methodenvergleich zeigten sich sehr gute Übereinstimmungen (Korrelationskoeffizient 0,995) zwischen der erfindungsgemäßen Methode und der Methode des Standes der Technik (Fig. 3.).

## Patentansprüche

1. Multivalentes Dextranreagenz zum Einsatz in Präzipitationstests zur Bestimmung einer spezifisch bindefähigen Substanz, bestehend aus Dextran, an das mehrere Moleküle eines Rezeptors R₁, der mit der zu bestimmenden Substanz spezifisch bindefähig ist, oder der spezifisch bindefähigen Substanz bzw. eines Analogons dieser Substanz gebunden sind.

2. Multivalentes Dextranreagenz gemäß Anspruch 1, dadurch gekennzeichnet, daß das Dextran ein Molekulargewicht von 10000 - 2000000 besitzt.

3. Multivalentes Dextranreagenz gemäß einem der Ansprüche 1 oder 2, dadurch gekennzeichnet, daß an Dextran 2 bis 50 Moleküle des Rezeptors R₁ oder der spezifisch bindefähigen Substanz bzw. des Analogons dieser Substanz gebunden sind.

4. Multivalentes Dextranreagenz gemäß einem der Ansprüche 1 -3, dadurch gekennzeichnet, daß die Bindung der Moleküle des Rezeptors R₁ oder der spezifisch bindefähigen Substanz bzw. des Analogons dieser Substanz an Dextran über miteinander bindefähige Rezeptoren R₃ und R₄ erfolgt, wobei R₃ an Dextran und R₄ an den Rezeptor R₁ oder die spezifisch bindefähige Substanz bzw. das Analogon dieser Substanz gebunden ist.

5. Multivalentes Dextranreagenz gemäß Anspruch 4, dadurch gekennzeichnet, daß R₃ Streptavidin und R₄ Biotin ist.

6. Multivalentes Dextranreagenz gemäß einem der Ansprüche 1 - 5, dadurch gekennzeichnet, daß der Rezeptor R₁ ein Antikörper oder ein mono- oder bivalentes Antikörperfragment ist.

7. Präzipitationstest zur Bestimmung einer spezifisch bindefähigen Substanz, dadurch gekennzeichnet, daß neben weiteren für das Verfahren notwendigen Reagenzien, wie Puffer, Reaktionsbeschleuniger, Entstörsubstanzen und/oder Detergenzien, ein multivalentes Dextranreagenz gemäß einem der Anspruche 1 - 6 eingesetzt wird.

8. Präzipitationstest gemaß Anspruch 7, dadurch gekennzeichnet, daß eine direkte Testführung benutzt wird.

9. Präzipitationstest gemäß Anspruch 7, dadurch gekennzeichnet, daß die kompetitive Testführung benutzt wird, wobei ein multivalentes Dextranreagenz, bestehend aus Dextran, an das mehrere Moleküle der spezifisch bindefähigen Substanz bzw. des Analogons dieser Substanz gebunden sind und zusätzlich ein mindestens bivalenter Rezeptor R₂, der mit der zu bestimmenden Substanz spezifisch bindefähig ist, eingesetzt werden.

10. Präzipitationstest gemäß Anspruch 9, dadurch gekennzeichnet, daß R₂ ein Antikörper oder ein bivalentes Antikörperfragment ist.

11. Präzipitationstest gemäß einem der Ansprüche 9 oder 10, dadurch gekennzeichnet, daß ein weiteres Dextranreagenz eingesetzt wird, bestehend aus Dextran, an das mehrere Moleküle des Rezeptors R₂ direkt gebunden sind.

12. Testkit zur Bestimmung einer spezifisch bindefähigen Substanz gemäß Anspruch 8, dadurch gekennzeichnet, daß er ein multivalentes Dextranreagenz enthält, bestehend aus Dextran, an das mehrere Moleküle des Rezeptors R₁, der mit der zu bestimmenden Substanz spezifisch bindefähig ist, oder der spezifisch bindefähigen Substanz bzw. eines Analogons dieser Substanz gebunden sind.

13. Testkit zur Bestimmung einer spezifisch bindefähigen substanz gemäß einem der Ansprüche 9 oder 10, dadurch gekennzeichnet, daß er ein multivalentes Dextranreagenz, bestehend aus Dextran, an das mehrere Moleküle der spezifisch bindefähigen Substanz bzw. eines Analogons dieser Substanz gebunden sind und einen mindestens bivalenten Rezeptor R₂, der mit der zu bestimmenden Substanz spezifisch bindefähig ist, enthält.

14. restkit zur Bestimmung einer spezifisch bindefähigen Substanz gemäß Anspruch 11, dadurch gekennzeichnet, daß er ein multivalentes Dextranreagenz, bestehend aus Dextran, an das mehrere Moleküle der spezifisch bindefähigen Substanz bzw. ein Analogon dieser Substanz gebunden sind und ein weiteres Dextranreagenz, bestehend aus Dextran, an das mehrere Moleküle des Rezeptors R₂ gebunden sind, enthält.

15. Verwendung eines multivalenten Dextranreagenzes gemäß einem der Ansprüche 1-6 in einem Präzipitationstest zur Bestimmung einer spezifisch bindefähigen Substanz.

16. Verwendung eines multivalenten Dextranreagenzes gemäß einem der Ansprüche 1-6 zur Herstellung eines Testkits gemäß einem der Ansprüche 12 - 14.

## Claims

1. Multivalent dextran reagent for use in precipitation tests for the determination of a specifically bindable substance comprising dextran to which several molecules of a receptor R₁ which is capable of specific binding to the substance to be determined or of the specifically bindable substance or of an analogue of this substance are bound.

2. Multivalent dextran reagent as claimed in claim 1, wherein the dextran has a molecular weight of 10000 - 2000000.

3. Multivalent dextran reagent as claimed in one of the claims 1 or 2, wherein 2 to 50 molecules of the receptor R₁ or of the specifically bindable substance or of the analogue of this substance are bound to dextran.

4. Multivalent dextran reagent as claimed in one of the claims 1 - 3, wherein the binding of the molecules of the receptor R₁ or of the specifically bindable substance or of the analogue of this substance to dextran is carried out via receptors R₃ and R₄ which are capable of binding to one another, whereby R₃ is bound to dextran and R₄ is bound to the receptor R₁ or to the specifically bindable substance or to the analogue of this substance.

5. Multivalent dextran reagent as claimed in claim 4, wherein R₃ is streptavidin and R₄ is biotin.

6. Multivalent dextran reagent as claimed in one of the claims 1 - 5, wherein the receptor R₁ is an antibody or a monovalent or bivalent antibody fragment.

7. Precipitation test for the determination of a specifically bindable substance, wherein a multivalent dextran reagent as claimed in one of the claims 1 - 6 is used in addition to further reagents which are necessary for the method such as buffers, reaction accelerators, substances which reduce interference and/or detergents.

8. Precipitation test as claimed in claim 7, wherein a direct test procedure is used.

9. Precipitation test as claimed in claim 7, wherein the competitive test procedure is used in which a multivalent dextran reagent comprising dextran to which several molecules of the specifically bindable substance or the analogue of this substance are bound and in addition a receptor R₂ which is at least bivalent and is capable of specific binding to the substance to be determined are used.

10. Precipitation test as claimed in claim 9, wherein R₂ is an antibody or a bivalent antibody fragment.

11. Precipitation test as claimed in one of the claims 9 or 10, wherein a further dextran reagent is used comprising dextran to which several molecules of the receptor R₂ are directly bound.

12. Test kit for the determination of a specifically bindable substance as claimed in claim 8, wherein it contains a multivalent dextran reagent comprising dextran to which several molecules of the receptor R₁ which is capable of specific binding to the substance to be determined or of the specifically bindable substance or of an analogue of this substance are bound.

13. Test kit for the determination of a specifically bindable substance as claimed in one of the claims 9 or 10, wherein it contains a multivalent dextran reagent comprising dextran to which several molecules of the specifically bindable substance or of an analogue of this substance are bound and a receptor R₂ which is at least bivalent and is capable of specific binding to the substance to be determined.

14. Test kit for the determination of a specifically bindable substance as claimed in claim 11, wherein it contains a multivalent dextran reagent comprising dextran to which several molecules of the specifically bindable substance or of an analogue of this substance are bound and a further dextran reagent comprising dextran to which several molecules of the receptor R₂ are bound.

15. Use of a multivalent dextran reagent as claimed in one of the claims 1 - 6 in a precipitation test for the determination of a specifically bindable substance.

16. Use of a multivalent dextran reagent as claimed in one of the claims 1 - 6 for the production of a test kit as claimed in one of the claims 12 - 14.

## Revendications

1. Réactif multivalent à base de dextrane, utilisé dans les tests de précipitation pour la détermination d'une substance capable de former une liaison spécifique, constitué par du dextrane auquel sont liées plusieurs molécules d'un récepteur R₁, lequel est capable de former une liaison spécifique avec la substance à déterminer, ou de la substance capable de former une liaison spécifique ou d'un analogue de cette substance.

2. Réactif multivalent à base de dextrane selon la revendication 1, caractérisé en ce que la masse moléculaire du dextrane est comprise entre 10 000 et 2 000 000.

3. Réactif multivalent à base de dextrane selon la revendication 1 ou 2, caractérisé en ce que 2 à 50 molécules du récepteur R₁ ou de la substance capable de former une liaison spécifique ou de l'analogue de cette substance sont liées au dextrane.

4. Réactif multivalent à base de dextrane selon l'une quelconque des revendications 1 à 3, caractérisé en ce que la liaison entre le dextrane et les molécules du récepteur R₁ ou de la substance capable de former une liaison spécifique ou de l'analogue de cette substance est réalisée par l'intermédiaire de récepteurs R₃ et R₄ capables de former une liaison l'un avec l'autre, R₃ étant lié au dextrane et R₄, au récepteur R₁ ou à la substance capable de former une liaison spécifique ou à l'analogue de cette substance.

5. Réactif multivalent à base de dextrane selon la revendication 4, caractérisé en ce que R₃ représente la streptavidine et R₄, la biotine.

6. Réactif multivalent à base de dextrane selon l'une quelconque des revendications 1 à 5, caractérisé en ce que le récepteur R₁ est un anticorps ou un fragment d'anticorps mono- ou bivalent.

7. Test de précipitation pour la détermination d'une substance capable de former une liaison spécifique, caractérisé en ce que, en plus d'autres réactifs nécessaires pour ce procédé, tels que des tampons, des accélérateurs de réaction, des substances antiperturbatrices et/ou des tensioactifs, on utilise un réactif multivalent à base de dextrane selon l'une quelconque des revendications 1 à 6.

8. Test de précipitation selon la revendication 7, caractérisé en ce que l'on utilise un mode de test direct.

9. Test de précipitation selon la revendication 7, caractérisé en ce que l'on utilise le mode de test par compétition, dans lequel on met en oeuvre un réactif multivalent à base de dextrane, constitué par du dextrane auquel sont liées plusieurs molécules de la substance capable de former une liaison spécifique ou de l'analogue de cette substance, et en plus un récepteur R₂ au moins bivalent, lequel est capable de former une liaison spécifique avec la substance à déterminer.

10. Test de précipitation selon la revendication 9, caractérisé en ce que R₂ est un anticorps ou un fragment d'anticorps bivalent.

11. Test de précipitation selon l'une quelconque des revendications 9 et 10, caractérisé en ce que l'on utilise un réactif supplémentaire à base de dextrane, constitué par du dextrane auquel sont directement liées plusieurs molécules de récepteur R₂.

12. Trousse d'analyse pour la détermination d'une substance capable de former une liaison spécifique selon la revendication 8, caractérisée en ce qu'elle contient un réactif multivalent à base de dextrane, constitué par du dextrane auquel sont liées plusieurs molécules de récepteur R₁, lequel est capable de former une liaison spécifique avec la substance à déterminer, ou de la substance capable de former une liaison spécifique ou d'un analogue de cette substance.

13. Trousse d'analyse pour la détermination d'une substance capable de former une liaison spécifique, selon la revendication 9 ou 10, caractérisée en ce qu'elle contient un réactif multivalent à base de dextrane, constitué par du dextrane auquel sont liées plusieurs molécules de la substance capable de former une liaison spécifique ou d'un analogue de cette substance, et un récepteur R₂ au moins bivalent, lequel est capable de former une liaison spécifique avec la substance à déterminer.

14. Trousse d'analyse pour la détermination d'une substance capable de former une liaison spécifique, selon la revendication 11, caractérisée en ce qu'elle contient un réactif multivalent à base de dextrane, constitué par du dextrane auquel sont liées plusieurs molécules de la substance capable de former une liaison spécifique ou d'un analogue de cette substance, et un autre réactif à base de dextrane, constitué par du dextrane auquel sont liées plusieurs molécules de récepteur R₂.

15. Utilisation d'un réactif multivalent à base de dextrane, selon l'une quelconque des revendications 1 à 6, dans un test de précipitation pour la détermination d'une substance capable de former une liaison spécifique.

16. Utilisation d'un réactif multivalent à base de dextrane selon l'une quelconque des revendications 1 à 6, pour la préparation d'une trousse d'analyse selon l'une quelconque des revendications 12 à 14.
